# EUROPEAN PATENT SPECIFICATION

(11) **EP 0 910 346 B1**
(45) Date of publication and mention of the grant of the patent: **17.03.2004**
(21) Application number: 97928185.4
(22) Date of filing: 10.06.1997
(51) Int. Cl.: A61K 9/20

(54) **MARKING RAPIDLY DISINTEGRATING DOSAGE FORMS**
MARKIEREN VON SCHNELL ZERFALLENDEN DOSIERUNGSFORMEN
MARQUAGE DE FORMES POSOLOGIQUES A DESINTEGRATION RAPIDE

(30) Priority: 17.06.1996 US 20274 P
(43) Date of publication of application: 28.04.1999
(73) Proprietor: JANSSEN PHARMACEUTICA N.V., 2340 Beerse (BE)
(72) Inventor: POSAGE, Gary, W., Ann Arbor, MI 48108 (US)
(74) Representative: Quaghebeur, Luc
(86) International application number: PCT/EP1997/003066
(87) International publication number: WO 1997/048384

(56) References cited:
- WO-A-93/23017
- WO-A-95/09608
- CH-A- 660 750
- US-A- 4 548 825
- PATENT ABSTRACTS OF JAPAN vol. 016, no. 380 (M-1295), 14 August 1992 & JP 04 122688 A (TAKEDA CHEM IND LTD), 23 April 1992,
- PATENT ABSTRACTS OF JAPAN vol. 010, no. 151 (M-483), 31 May 1986 & JP 61 005988 A (TOSHIBA KK), 11 January 1986,

## Description

The present invention relates to a method of marking the surface of solid rapidly disintegrating dosage forms comprising a non-compressed, porous network of matrix forming materials manufactured by a process including a lyophilization step or a solid state dissolution step of a frozen mixture of a solvent with matrix forming materials, characterized in that legible marks and/or characters are applied to the surface of said solid rapidly disintegrating dosage forms using laser imprinting or ink-jet printing. The present also relates to a solid rapidly disintegrating dosage form with legible marks obtainable by ink jet printing a non-compressed, porous network of matrix forming materials manufactured by a process including a lyophilization step or a solid state dissolution step of a frozen mixture of a solvent with matrix forming materials.

Solid rapidly disintegrating dosage forms loaded with a predetermined quantity of an active ingredient are known from GB-A-1,548,022 (US-4,305,502). These solid dosage forms comprise a porous network of a matrix material carrying an active ingredient, the matrix material consisting of a water-soluble or a water-dispersable carrier material. The solid dosage forms are prepared by freeze-drying or lyophilization of the solvent from a frozen solution or suspension of the matrix material and the active ingredient.

Various improvements for preparing dosage forms by lyophilization have been developed. GB-A-2,111,423 and US-4,371,516 disclose such methods of preparing solid dosage forms which are rapidly disintegrated by water and in which a network of matrix material carries a predetermined amount of an active ingredient, particularly a pharmaceutical substance. Such dosage forms find many applications, particularly where it is desired to administer, dispense or otherwise utilise an active ingredient in predetermined unit quantities. For example, certain active ingredients which are used in solution or suspension form, but which are difficult or hazardous to transport or store in such form, may be converted into a solid form which can be added by the user to an aqueous medium to produce the desired solution or dispersion containing a predetermined amount of the active ingredient. Further, the active ingredient may be a reagent which can be added to a known amount of aqueous liquid to produce a standardised liquid composition which then can be used, for example, in chemical analysis. Further, the active ingredient may be a diagnostic compound which has to be added to a biological sample (e.g. blood, urine) and thus allows one to determine the amount of a particular constituent present in the sample. Preferably, however, the active ingredient is a drug substance for human or veterinary use. Rapidly dissolving solid drug dosage forms are particularly suitable for oral administration. When orally administered they generally disintegrate rapidly in the mouth (e.g. within one or two seconds) and thus the dosage form is a particularly advantageous means for administering drugs to humans and animals. Such dosage forms can be used as alternatives to conventional tablets, pills or capsules, particularly for patients - humans and animals alike - who have difficulty in swallowing these conventional dosage forms.

US-4,642,903 teaches a procedure for preparing a freeze-dried foam dosage form using conventional lyophilization techniques which results in rapidly dissolving pharmaceutical dosage forms.

WO-93/23017 addresses a problem intrinsic to conventional lyophilization methods namely the lack of uniform porosity in the lyophilized product. Uniform porosity in a lyophilized product is critical for post-loading a placebo or unloaded dosage form with an active ingredient. WO-93/23017 concerns a method of producing a dosage form that will avoid cracking and meltback, has adequate strength, and porosity and exhibits a fast speed of dissolution.

Other methods for the preparation of solid dosage forms which rapidly disintegrate in the mouth, namely solid state dissolution techniques are disclosed in US-5,039,540, US-A-5,215,756, US-A-5,330,764, and US-5,298,261.

Solid dosage forms as provided by the prior art are used to deliver predetermined amounts of active ingredients. Since the administration of such products is associated with many risks, there is a need to bestow an identity on them. Such risks include, for example, errors in the administration of medicines by physicians, pharmacists or by the end-users, the patients. Several regulatory authorities have issued regulations requiring all solid oral dosage forms to bear a code identifying the product and the holder of the manufacturing authorisation.

Besides using distinct colours and dosage shapes, this problem can be solved satisfactorily in classic solid dosage forms such as compressed tablets and capsules, by imprinting. Typically, a mark or text is printed on the dosage form surface, e.g. a company logo or name, a product name, a trade mark, or a number indicating the amount of active ingredient in the dosage form. An alternative solution applicable to tablets consists of intagliating the surface thereof by a compression punching, incision or engraving procedure. The intagliations can be highlighted by filling with an optically anisotropic substance (e.g. EP-0,060,023 and EP-0,096,982), by dry filling with a material having a different colour (EP-0,088,556) or by wet filling with a material having a different colour (EP-0,501,553).

The use of distinct colours and dosage shapes clearly also applies to solid rapidly dissolving dosage forms comprising a non-compressed porous network of matrix forming materials. Thus far, however, no solutions have been offered for marking such dosage forms with imprints or intagliations. The reduced mechanical strength, in particular the compressible nature of these dosage forms, as compared to conventional compressed tablets, has impeded the application of prior art printing and intagliating techniques thereto. Said techniques rely on physical contact and pressure between the dosage form and the printing mat or stamp to transfer the mark to the tablet.

Now, a method has been developed of marking the surface of solid rapidly disintegrating dosage forms comprising a non-compressed, porous network of matrix forming materials, in which the foregoing problem is solved by applying legible marks and / or characters to the surface of said solid rapidly disintegrating dosage forms using non-contact marking techniques.

The present invention provides two such non-contact marking methods, namely laser imprinting and ink-jet printing.

According to the first method a laser is used to etch marks and / or characters into the surface of the dosage form by vaporizing a shallow and narrow portion of the surface. In order for the marks and or characters to be legible it suffices that a shadow be created in the valley left by the impinging laser beam. Since laser beams typically have a very small beam divergence, typically in the order of a few milliradian, the breadth of the valley at the surface of the solid dosage form can be very narrow, indeed. Under these circumstances, even a very shallow valley will cast a visible shadow. Most importantly, since the solid rapidly dissolving dosage forms comprise a porous network of matrix forming materials, very little material has to be vaporized in order to attain the desired objective. This is of great practical value for several reasons. First, since the energy input required to vaporize solid material is proportional to the amount of said material, little energy will be needed in the vaporization process. In effect, this means that the individual dosage forms will have to be irradiated only very shortly, or conversely that a great many dosage forms can be etched per time unit. This feature of high-throughput is of prime importance in rendering the marking method industrially viable. Secondly, the amount of material removed from the solid dosage form, in particular the amount of active ingredient removed from it, is so small relative to the total amount of the dosage form as to be negligable. Finally, the method using a laser has the further advantage of not introducing any new materials into the dosage form, which is a distinct advantage in pharmaceutical applications.

A first mode of etching the marks and / or characters into the surface of the dosage form comprises passing the laser beam through a mask or stencil, preferably in metal, precut with the desired information. The laser beam forms an image of the mask and is then directed onto the surface of the part of the solid dosage form to be marked. Suitable lasers are those which have been designed for industrial marking applications and which produce short, powerful pulses of light energy, preferably in the infrared range (λ = 1 µm - 1 mm), e.g. carbon dioxide lasers (λ = 10.6 µm), helium-neon lasers (λ = 3.39 µm) and the like. Preferably the pulsed lasers are used at low pulse energy (a few Joules) and at high repetition rate (e.g. 20 Hz) in order to attain a high, industrially viable mark speed (e.g. 1200 marks/min).

An alternative comprises moving a solid dosage form past a plurality of pulsed laser beams arranged along a single line thus creating dot matrix patterns on the surface of the solid dosage form.

A third mode of etching the marks and / or characters into the surface of the dosage form comprises scanning a laser beam over the surface of the dosage form using rapidly rotating, computer controlled mirrors. In this mode, a YAG (yttrium aluminum garnet) or continuous wave carbon dioxide laser is used preferably to etch the product code inforrmation on the surface of a solid dosage form.

The vaporized portion of the surface comprises degraded matrix forming materials which are advantageously removed by suction using art-known devices.

According to the second method an ink-jet printer can be used to print marks and / or characters onto the surface of the dosage form by dotting droplets of an ink onto said surface. Prior art methods of ink-jet printing, according to the inventor's knowledge, thus far have been applied exclusively to the marking of articles that are substantially impermeable to the ink. Surprisingly, it has proven perfectly possible to extend the ink-jet printing technology to highly porous solid dosage forms.

The ink used to mark dosage forms must be suspended or dissolved in a liquid, volatile carrier such as water, alcohol, or mixtures thereof. Water based ink jet inks can contain from 70 to 90 % water depending on the nature of the ink. Because of the small droplet size, the solvent evaporates rapidly and does not affect the structure of the solid dosage form. The ink can be edible or non-edible depending on the final use of the dosage form. In case the dosage form comprises a drug for human or veterinary use, obviously only pharmaceutically acceptable, edible inks can be used in the present method of marking the dosage form by means of an ink-jet printer.

The above methods are particularly useful when the dosage form is loaded with an active ingredient and is shaped as a tablet. The methods are more particularly suitable when the active ingredient is a drug substance for human or veterinary use and the solid rapidly disintegrating dosage form is a pharmaceutical tablet for oral administration.

The dosage form comprises a porous network of matrix forming materials :
i) a water-soluble, hydratable gel or foam-forming material,
ii) a rigidifying agent for the gel or foam-forming material, and optionally
iii) one or more amino acids.

The solid dosage forms are prepared by lyophilization or by a solid state dissolution technique of a frozen mixture of a solvent with the matrix forming materials. These mixtures may be in a variety of forms such as solutions, suspensions, dispersions, emulsions, foams. Persons skilled in the art will recognize acceptable methods for preparing each of these.

Water is preferably employed as the solvent in the composition which is frozen and desolvated. An additional co-solvent (such as an alcohol) may also be used if it is desired to improve the solubility, dispersability or wettability of any of the ingredients of the composition.

Suitable water-soluble, hydratable gel or foam-forming materials include proteinaceous materials such as gelatin, gelatin A, gelatin B, fluid gelatin, modified fluid gelatin, gelatin derivatives, albumin, soy fiber protein, wheat and psyllium seed proteins, potato protein, papain; phospholipids such as coacervate egg lecithin, or lecithin; gums such as acacia, guar, agar, locust bean, xanthan and tragacanth gum ; polysaccharides such as alginates (polymannuronic acid), chitosan, carrageenans, dextrans, dextrins, maltrins (maltodextrins), pectins (polygalacturonic acid), microcrystalline cellulose, corn syrup solids, konjac flour, rice flour, wheat gluten ; synthetic polymers such as polyvinylpyrrolidone, sodium carboxymethylcellulose, sodium starch glycolate, hydroxyethylcellulose; and polypeptide/protein or poly-saccharide complexes such as gelatin-acacia complexes, each singly or in combination.

Suitable rigidifying agents include monosaccharides, linear and cyclic oligosaccharides and polysaccharides, e.g. mannitol, xylitol, sorbitol, dextrose, fructose, sucrose, lactose, maltose, galactose, trehalose ; cyclic sugars such as cyclodextrins e.g. beta-cyclodextrin and 2-hydroxypropyl-beta-cyclodextrin ; dextran, dextrin ; and further include inorganic substances such as sodium phosphate, sodium chloride, magnesium aluminum silicates, magnesium trisilicate, natural clays, or a combination thereof. The preferred rigidifying agent is mannitol.

Suitable amino acids have from 2 to 12 carbon atoms, e.g. glycine, L-alanine, L-aspartic acid, L-glutamic acid, L-hydroxyproline, L-isoleucine, L-leucine, L-phenylalanine, or a combination thereof. Glycine is the preferred amino acid. Dosage forms containing glycine as one of the matrix forming components have several advantages : quick dissolution and disintegration in aqueous media, pleasant taste and mouthfeel, nutritional value, low caloric content and noncariogenicity. Of particular importance is the fact that these dosage forms can be produced with minimal cracking or meltback and that they have uniform porosity and adequate strength of handling, i.e. resistance to disintegration or crumbling under normal manufacturing and handling conditions. These latter properties contribute to the feasibility of the post-loading processes whereby active ingredients are loaded onto placebo or unloaded dosage forms.

Preferred matrix forming agents include pharmaceutical grade gelatins, pectins (nonhydrolyzed, partially hydrolyzed or hydrolyzed), glycine and mannitol. A particularly preferred combination of matrix forming agents comprises gelatin, glycine and mannitol.

The percentages and ratios mentioned in the following paragraphs are all by weight.

The solution or dispersion of materials for preparing the matrix can contain from 0.1 % to 15 % by weight of gel or foam forming material, in particular from 1 % to 5 % more in particular from 1.2 % to 3 %. It can further contain from 0.5 % to 10 %, in particular from 0.8 % to 2.5 % by weight of amino acid and from 0.5 % to 10 %, in particular from 1 % to 4 % of rigidifying agent, the remainder being solvent and secondary components as mentioned hereinafter.

The ratios between these materials may vary within certain ranges. In particular the weight by weight ratio of the total amount of amino acids to that of the water-soluble, hydratable gel or foam-forming material is from 1 : 1 to 1 : 3. A preferred ratio is 1.5 : 1. The weight by weight ratio of the amount of the water-soluble, hydratable gel or foam-forming material to that of the rigidifying agent is from 2 : 1 to 1 : 2 . A preferred ratio is 1.5 : 2.

Typically, the weight by weight ratio of the total amount of non-solvent components to that of the water in the aqueous composition is in the range of about 1 : 9 to 1 : 33, in particular from about 1 : 13 to 1 : 30, for example about 1 : 20.

Solid rapidly dissolving dosage forms find many applications, particularly where it is desired to administer, dispense or otherwise utilise an active ingredient in predetermined unit quantities. The active ingredient in particular is a drug substance for human of veterinary use.

The active ingredient used in the solid rapidly dissolving dosage form may be present in a coated form. For example, it may be present in particulate form and the particles of the active ingredient may be coated with an appropriate coating agent so as to protect it from process diluents, the aqueous environment of the suspension or of the oral or other mucosal cavity, or other environmental conditions that would dissolve or deteriorate said active ingredient. These coating materials may be selected from natural or synthetic polymers that are either hydrophilic or hydrophobic in nature or other hydrophobic materials such a fatty acid, glycerides, triglycerides and mixtures thereof. In this way, the taste of the active or bioactive agent may be masked, while at the same time permitting the solid dosage form to dissolve rapidly upon contact with physiological diluents. Examples of bitter active ingredients that may be coated in accordance with the present invention include acetaminophen, ibuprofen, chlorpheniramine maleate, pseudo-ephedrine, dextromethorphan, cisapride, domperidone, risperidone. Pharmaceutical applications comprise dosage forms having mucoadhesive properties or designed to deliver a drug at a controlled rate; dosing units designed to deliver drugs in the eye, in vaginal, rectal and other body orifices; solid dosage forms designed to replace liquid formulations; dry medicated preparations for topical application after resolvation (reconstitution); preparation of medicated units or sheets for topical application; preparation of more palatable dosage forms of drugs that exhibit disagreeable organoleptic properties; dosage forms for oral delivery of drugs to persons who have difficulty swallowing tablets or capsules.

Secondary components such as nutrients, vitamins, other active ingredients, sweeteners, flavouring agents, colouring agents, surfactants, preservatives, antioxidants, viscosity enhancers, minerals, diagnostics, fertilizers and insecticides may also be incorporated in the formulation of the dosage form.

The solution or suspension of which the dosage forms are made may further contain the secondary components mentioned before. Xanthan gum or polyacrylic acid polymers and salts thereof (also referred to as carbomers or carboxyvinyl polymers, e.g. Carbopol™) may be added in order to increase viscosity, or to keep the components of the mixture in suspension.

The aqueous compositions may be frozen by any conventional cooling process. For example, the mixture may be frozen by dispensing it into preformed molds corresponding to the size and shape of the desired dosage form and subsequently cooling such molds on refrigerated shelves or in refrigerated chambers. Alternatively, the molds containing the mixture may be passed through a stream of cold gas or vapor, such as liquid nitrogen in a freezing tunnel. In a preferred method of freezing, the composition is passed through a freezing tunnel into which liquid nitrogen is injected, the liquid nitrogen being vaporised and the resulting cold gaseous nitrogen being passed over the composition. Another method for frreezing the mixtures in the molds is to surround the molds in dry ice until the mixture has frozen.

The best-known process of removing solvents from frozen solutions or dispersions is lyophilization which involves desolvation of the mixture by sublimation of the solvent under a vacuum. If desired, the frozen compositions may be stored in a cold store before the sublimation process is carried out. The sublimation may be carried out in a freeze drier by subjecting the frozen composition in the mold to reduced pressure and, if desired, controlled application of heat to aid the sublimation. The pressure can be below 4 mmHg (533 Pa), e.g. below 0.3 mmHg (40 Pa), for example 0.1 to 0.2 mmHg (13.3 to 26.6 Pa) or even below 0.05 mmHg (6.7 Pa). The initial temperature in the freeze drier may be, for example, as high as 60°C and this temperature can be reduced (e.g. to 40°C) as the temperature of the frozen composition increases. Various methods and improvements are described in the references cited at the very beginning of the specification. The frozen compositions also may be removed from the mold prior to lyophilization.

The dosage forms can also be prepared by a solid-state dissolution method of removing solid solvent from solidified samples. In this less conventional method, one or more delivery matrix forming agents are dissolved or dispersed in a first solvent, frozen and subsequently contacted with a second solvent at a temperature at or higher than the solidification point of the second solvent and at a temperature at or lower than the solidification point of the first solvent. The first solvent in the solidified state is substantially miscible with the second solvent, while the matrix forming agent(s) are substantially insoluble in the second solvent. The first solvent is thereby substantially removed from the solidified matrix yielding a solid matrix substantially free of the first solvent. Typically, the first solvent is water and the second ethanol.

The mold can be, for example a depression in a metal plate (e.g. an aluminium plate). The plate may contain more than one depression, each depression being of the size and shape corresponding to the desired size of the shaped article. However the mold may also be a depression in a sheet of filmic material. The filmic material may contain more than one depression. The filmic material may be similar to that employed in conventional blister packs which are used for packaging pharmaceutical tablets and like medicament forms. For example, the filmic material may be made of thermoplastic material with the depressions formed by thermo-forming. The preferred filmic material is a talc-filled polypropylene film or a polyvinyl chloride film. Laminates of filmic material such as polyvinyl chloride/polyvinylidene chloride, polyvinyl chloride / polytetrafluorethylene or polyvinyl chloride / polyvinylidene chloride/polyethylene may also be used.

Where lyophilization is used, it may be advantageous to freeze the matrix material solution in molds that are coated or lined for easy release of the frozen material. Preferred molds are thermoformed cups made in talc-filled polypropylene sheets, optionally siliconized with a layer of silicone/simethicone baked on the surface(s) which come into contact with the aqueous composition.

Dosage forms may be prepared in a wide variety of sizes, ranging from about 0.25 ml or g to 30 ml or g and larger. Large dosage forms may be advantageously prepared by the solid state dissolution process without the long drying times required by lyophilization. The frozen and desolvated dosage forms may be of a size corresponding to the desired size of two or more dosage forms. For example, the composition may be frozen in a tray and the solvent removed from the frozen composition to produce a slab or a sheet of desolvated product corresponding in size to that of a number of the desired shaped articles. The sheet may be subdivided to form products of the desired size and the active ingredient may be post-loaded on to the subdivided products by injecting a predetermined amount of a suspension comprising said active ingredient. A particular advantage of this alternative resides in the fact that the subdivision of the sheet does not need to be carried out accurately since a measured amount of active ingredient is added to the subdivided products. In addition, if the injected suspension does not diffuse excessively through the sheet of sublimed product, the sheet may be dosed with the predetermined amount of active ingredient at selected positions on the sheet prior to subdivision and the sheet subsequently subdivided to give dosage forms each containing the predetermined amount of active ingredient.

The present invention also provides marked, solid rapidly disintegrating dosage forms obtainable by any one of the processes described hereinbefore.

The speed with which the marked tablet prepared by the inventive method disintegrates is dependent entirely or at least in large part on the choice of matrix forming agent(s), their concentration and the solidification/desolvation process conditions. In particular, dosage forms of the size mentioned in the examples described hereinafter, will dissolve or disperse rapidly, for example, in less than about 10 seconds and generally faster e.g. in less than about 5 seconds or even less, e.g. within 1 to 2 seconds.

The dosage forms disperse rapidly in water, e.g. in less than 10 seconds. The disintegration time of a dosage form is determined to check whether it is capable of being disintegrated by water sufficiently rapidly using a standard tablet disintegration apparatus as described in British Pharmacopoeia, 1980, Vol II, Appendix XII A, but with the standard 2.00 mm wire mesh replaced by stainless steel 40 mesh screen. A sample product is placed in a dry tube held above the surface of the water. The apparatus is started and the sample immersed in water at 20°C. The sample should disperse on the liquid surface and any solid residue should pass through the 40 mesh screen within 10 seconds, preferably within 5 seconds and ideally within 1 to 2 seconds.

## Claims

1. A method of marking the surface of solid rapidly disintegrating dosage forms comprising a non-compressed, porous network of matrix forming materials manufactured by a process including a lyophilization step or a solid state dissolution step of a frozen mixture of a solvent with matrix forming materials, **characterized in that** legible marks and / or characters are applied to the surface of said solid rapidly disintegrating dosage forms using laser imprinting or ink-jet printing.

2. A method according to claim 1 wherein a laser is used to etch marks and / or characters into the surface of the dosage form by vaporizing a shallow and narrow portion of the surface.

3. A method according to claim 2 wherein the marks and / or characters are etched into the surface of the dosage form by passing the laser beam through a precut mask or stencil.

4. A method according to claim 2 wherein the marks and / or characters are etched into the surface of the dosage form by moving the dosage form past a plurality of pulsed laser beams arranged along a single line, thus creating dot matrix patterns on the surface.

5. A method according to claim 2 wherein the marks and / or characters are etched into the surface of the dosage form by scanning a laser beam over the surface of the dosage form using rapidly rotating, computer controlled mirrors.

6. A method according to claim 1 wherein an ink-jet printer is used to print marks and / or characters onto the surface of the dosage form by dotting droplets of an ink onto said surface.

7. A method according to claim 6 wherein the ink is suspended or dissolved in a liquid, volatile carrier.

8. A method according to claim 7 wherein the dosage form is marked by means of an ink-jet printer dotting droplets of a pharmaceutically acceptable, edible ink onto the surface of the dosage form.

9. A method according to claim 1 wherein the dosage form is loaded with a predetermined amount of an active ingredient and is shaped as a tablet.

10. A method according to claim 9 wherein the active ingredient is a drug substance for human or veterinary use and the solid rapidly disintegrating dosage form is a pharmaceutical tablet for oral administration.

11. A method according to claim 1 wherein the matrix forming material comprises
i) a water-soluble, hydratable gel or foam-forming material,
ii) a rigidifying agent for the gel or foam-forming material, and optionally
iii) one or more amino acids.

12. A method according to claim 11 wherein the gel or foam-forming material is a proteinaceous materials such as gelatin, gelatin A, gelatin B, fluid gelatin, modified fluid gelatin, gelatin derivatives, albumin, soy fiber protein, wheat and psyllium seed proteins, potato protein, papain ; phospholipids such as coacervate egg lecithin, or lecithin; gums such as acacia, guar, agar, locust bean, xanthan and tragacanth gum ; polysaccharides such as alginates (polymannuronic acid), chitosan, carrageenans, dextrans, dextrins, maltrins (maltodextrins), pectins (polygalact-uronic acid), microcrystalline cellulose, corn syrup solids, konjac flour, rice flour, wheat gluten ; synthetic polymers such as polyvinylpyrrolidone, sodium carboxymethyl-cellulose, sodium starch glycolate, hydroxyethylcellulose; and polypeptide/protein or polysaccharide complexes such as gelatin-acacia complexes, each singly or in combination.

13. A method according to claim 11 wherein the rigidifying material is a monosaccharide, a linear or cyclic oligosaccharide, a polysaccharide, or an inorganic substance, or a combination thereof.

14. A method according to claim 13 wherein the rigidifying material is mannitol, xylitol, sorbitol, dextrose, fructose, sucrose, lactose, maltose, galactose, trehalose ; a cyclic sugar such as beta-cyclodextrin or 2-hydroxypropyl-beta-cyclodextrin, dextran, dextrin, an inorganic substance such as sodium phosphate, sodium chloride, a magnesium aluminum silicate, magnesium trisilicate, a natural clay, or a combination thereof.

15. A method according to claim 11 wherein the amino acid is glycine, L-aspartic acid, L-glutamic acid, L-hydroxyproline, L-isoleucine, L-phenylalanine, or a combination thereof.

16. A method according to claim 11 wherein the matrix forming materials further comprise nutrients, vitamins, other active ingredients, sweeteners, flavouring agents, colouring agents, surfactants, preservatives, antioxidants, viscosity enhancers, minerals, diagnostics, fertilizers or insecticides.

17. A solid rapially disintegrating dosage form with legible marks and/or characters obtainable by ink-jet printing as claimed in claims 1, 6 to 16.

18. A dosage form according to claim 17 which disintegrates within 10 seconds in water at 20°C.

## Patentansprüche

1. Verfahren zur Markierung der Oberfläche von festen, schnell zerfallenden Dosierungsformen, bestehend aus einem nichtverpreßten, porösen Netzwerk matrixbildender Materialien, hergestellt durch ein Verfahren, das einen Lyophilisierungsschritt oder einen Festphasenlösungsschritt einer gefrorenen Mischung aus einem Lösungsmittel und matrixbildenden Materialien umfaßt, **dadurch gekennzeichnet, daß** lesbare Markierungen und/oder Schriftzeichen unter Anwendung eines Laserdruckverfahrens oder eines Tintenstrahldruckverfahrens auf die Oberfläche der festen, schnell zerfallenden Dosierungsformen aufgebracht werden.

2. Verfahren nach Anspruch 1, wobei ein Laser zum Ätzen von Markierungen und/oder Schriftzeichen in die Oberfläche der Dosierungsform verwendet wird, wobei ein flacher und enger Teil der Oberfläche verdampft wird.

3. Verfahren nach Anspruch 2, wobei die Markierungen und/oder Schriftzeichen in die Oberfläche der Dosierungsform geätzt werden, indem man den Laserstrahl durch eine zurechtgeschnittene Maske bzw. Schablone führt.

4. Verfahren nach Anspruch 2, wobei die Markierungen und/oder Schriftzeichen in die Oberfläche der Dosierungsform geätzt werden, indem man die Dosierungsform an mehreren gepulsten Laserstrahlen vorbeiführt, die entlang einer einzigen Linie angeordnet sind, wodurch auf der Oberfläche Punktmatrixmuster erzeugt werden.

5. Verfahren nach Anspruch 2, wobei die Markierungen und/oder Schriftzeichen in die Oberfläche der Dosierungsform geätzt werden, indem man die Oberfläche der Dosierungsform unter Anwendung schnell rotierender, computergesteuerter Spiegel mit einem Laserstrahl abtastet.

6. Verfahren nach Anspruch 1, bei dem man einen Tintenstrahldrucker zum Aufdrucken von Markierungen und/oder Schriftzeichen auf die Oberfläche der Dosierungsform verwendet, indem man Tröpfchen einer Tinte auf die Oberfläche aufpunktet.

7. Verfahren nach Anspruch 6, wobei die Tinte in einem flüssigen, flüchtigen Träger suspendiert oder gelöst ist.

8. Verfahren nach Anspruch 7, wobei die Dosierungsform mit einem Tintenstrahldrucker markiert wird, der Tröpfchen einer pharmazeutisch unbedenklichen, eßbaren Tinte auf die Oberfläche der Dosierungsform aufpunktet.

9. Verfahren nach Anspruch 1, wobei die Dosierungsform eine vorbestimmte Menge eines Wirkstoffs enthält und als Tablette geformt ist.

10. Verfahren nach Anspruch 9, wobei es sich bei dem Wirkstoff um eine Arzneimittelsubstanz für die Anwendung bei Menschen oder Tieren handelt und es sich bei der festen, schnell zerfallenden Dosierungsform um eine pharmazeutische Tablette für die orale Verabreichung handelt.

11. Verfahren nach Anspruch 1, wobei das matrixbildende Material
i) ein wasserlösliches, hydratisierbares Gel oder schaumbildendes Material,
ii) ein Versteifungsmittel für das Gel bzw. das schaumbildende Material und gegebenenfalls
iii) eine oder mehrere Aminosäuren
enthält.

12. Verfahren nach Anspruch 11, wobei es sich bei dem Gel bzw. dem schaumbildenden Material um ein proteinartiges Material wie Gelatine, Gelatine A, Gelatine B, Flüssiggelatine, modifizierte Flüssiggelatine, Gelatinderivate, Albumin, Sojafaserprotein, Weizen- und Flohsamenproteine, Kartoffelprotein und Papain, Phospholipide wie Koacervat-Eierlicithin oder Lecithin, Gummis wie Gummi arabicum, Guargummi, Agargummi, Johannesbrotgummi, Xanthangummi und Traganthgummi, Polysaccharide wie Alginate (Polymannuronsäure), Chitosan, Carrageenane, Dextrane, Dextrine, Maltrine (Maltodextrine), Pectine (Polygalacturonsäure), mikrokristalline Cellulose, feste Maissirupbestandteile, Konjakmehl, Reismehl und Weizenkleber, synthetische Polymere wie Polyvinylpyrrolidon, Natriumcarboxymethylcellulose, Natriumstärkeglycolat und Hydroxyethylcellulose und Polypeptid/Protein- oder Polysaccharidkomplexe wie Gelatine/Gummi-arabicum-Komplexe, jeweils einzeln oder kombiniert, handelt.

13. Verfahren nach Anspruch 11, wobei es sich bei dem Versteifungsmaterial um ein Monosaccharid, ein geradkettiges oder cyclisches Oligosaccharid, ein Polysaccharid oder eine anorganische Substanz oder eine Kombination davon handelt.

14. Verfahren nach Anspruch 13, wobei es sich bei dem Versteifungsmaterial um Mannit, Xylit, Sorbit, Dextrose, Fructose, Saccharose, Laktose, Maltose, Galactose, Trehalose, einen cyclischen Zucker wie beta-Cyclodextrin oder 2-Hydroxypropyl-beta-cyclodextrin, Dextran, Dextrin, eine anorganische Substanz wie Natriumphosphat, Natriumchlorid, ein Magnesiumalumosilikat, Magnesiumtrisilikat, einen natürlichen Ton oder eine Kombination davon handelt.

15. Verfahren nach Anspruch 11, wobei es sich bei der Aminosäure um Glycin, L-Asparaginsäure, L-Glutaminsäure, L-Hydroxyprolin, L-Isoleucin, L-Phenylalanin oder eine Kombination davon handelt.

16. Verfahren nach Anspruch 11, wobei die matrixbildenden Materialien weiterhin Nährstoffe, Vitamine, andere Wirkstoffe, Süßstoffe, Geschmacksstoffe, Farbstoffe, Tenside, Konservierungsstoffe, Antioxidationsmittel, Viskositätverbesserer, Mineralien, Diagnostika, Düngemittel oder Insektizide enthalten.

17. Feste, schnell zerfallende Dosierungsform mit lesbaren Markierungen und/oder Schriftzeichen, erhältlich durch ein Tintenstrahldruckverfahren nach den Ansprüchen 1 und 6 bis 16.

18. Dosierungsform nach Anspruch 17, die in Wasser bei 20°C innerhalb von 10 Sekunden zerfällt.

## Revendications

1. Méthode de marquage de la surface de formes posologiques solides à désintégration rapide comprenant un réseau poreux, non comprimé, de matières formant une matrice, fabriquées par un procédé intégrant une étape de lyophilisation ou une étape de dissolution à l'état solide d'un mélange congelé d'un solvant et de matières formant une matrice, **caractérisée en ce que** des marques et / ou des caractères lisibles sont appliqués à la surface desdites formes posologiques solides à désintégration rapide par impression laser ou par impression à jet d'encre.

2. Méthode selon la revendication 1, dans laquelle un laser est utilisé pour graver des marques et / ou des caractères dans la surface de la forme posologique par vaporisation d'une partie peu profonde et étroite de la surface.

3. Méthode selon la revendication 2, dans laquelle les marques et / ou les caractères sont gravés dans la surface de la forme posologique par passage du faisceau laser à travers un masque ou un stencil prédécoupé.

4. Méthode selon la revendication 2, dans laquelle les marques et / ou les caractères sont gravés dans la surface de la forme posologique par passage de la forme posologique devant de multiples faisceaux laser pulsés disposés le long d'une seule ligne, en créant ainsi des tracés sous forme de matrices de points sur la surface.

5. Méthode selon la revendication 2, dans laquelle les marques et / ou les caractères sont gravés dans la surface de la forme posologique par balayage de la surface de la forme posologique avec un faisceau laser en utilisant des miroirs à rotation rapide contrôlés par ordinateur.

6. Méthode selon la revendication 1, dans laquelle une imprimante à jet d'encre est utilisée pour imprimer des marques et / ou des caractères sur la surface de la forme posologique en déposant point par point des gouttelettes d'une encre sur ladite surface.

7. Méthode selon la revendication 6, dans laquelle l'encre est en suspension ou dissoute dans un support liquide volatil.

8. Méthode selon la revendication 7, dans laquelle la forme posologique est marquée au moyen d'une imprimante à jet d'encre déposant point par point des gouttelettes d'une encre comestible, acceptable au plan pharmaceutique, sur la surface de la forme posologique.

9. Méthode selon la revendication 1, dans laquelle la forme posologique est chargée avec une quantité prédéterminée d'un principe actif et se présente sous la forme d'un comprimé.

10. Méthode selon la revendication 9, dans laquelle le principe actif est une substance médicamenteuse à usage humain ou vétérinaire et la forme posologique solide à désintégration rapide est un comprimé pharmaceutique destiné à une administration orale.

11. Méthode selon la revendication 1, dans laquelle la matière formant une matrice comprend :
i) un gel ou une matière formant de la mousse, hydrosoluble et hydratable,
ii) un agent rigidifiant pour le gel ou la matière formant de la mousse, et facultativement
iii) un ou plusieurs acides aminés.

12. Méthode selon la revendication 11, dans laquelle le gel ou la matière formant de la mousse est une matière protéinique comme la gélatine, la gélatine A, la gélatine B, la gélatine liquide, la gélatine liquide modifiée, les dérivés de la gélatine, l'albumine, les protéines de fibre de soja, les protéines de blé et de graines de psyllium, les protéines de pomme de terre, la papaïne ; des phospholipides comme le coacervat de lécithine d'oeuf, ou la lécithine ; des gommes comme la gomme acacia, la gomme de guar, l'agar-agar, la gomme de caroube, la gomme xanthane et la gomme adragante ; des polysaccharides comme les alginates (acide polymannuronique), le chitosane, les carraghénanes, les dextranes, les dextrines, les maltrines (maltodextrines), les pectines (acide polygalacturonique), la cellulose microcristalline, les solides de sirop de maïs, 1a farine de konjac, la farine de riz, le gluten de blé ; des polymères de synthèse comme le polyvinylpyrrolidone, la carboxyméthylcellulose sodique, le glycolate d'amidon sodique, l'hydroxyéthylcellulose ; et des complexes polypeptide/protéine ou des complexes de polysaccharides comme les complexes gélatine-acacia, chacun seul ou en combinaison.

13. Méthode selon la revendication 11, dans laquelle la matière rigidifiante est un monosaccharide, un oligosaccharide linéaire ou cyclique, un polysaccharide, ou une substance inorganique, ou une combinaison de ceux-ci.

14. Méthode selon la revendication 13, dans laquelle la matière rigidifiante est le mannitol, le xylitol, le sorbitol, le dextrose, le fructose, le saccharose, le lactose, le maltose, le galactose, le tréhalose ; un sucre cyclique comme la bêta-cyclodextrine ou la 2-hydroxypropyl-bêta-cyclodextrine, le dextrane, la dextrine, une substance inorganique comme le phosphate de sodium, le chlorure de sodium, un silicate de magnésium et d'aluminium, un trisilicate de magnésium, une argile naturelle, ou une combinaison de ceux-ci.

15. Méthode selon la revendication 11, dans laquelle l'acide aminé est la glycine, l'acide L-aspartique, l'acide L-glutamique, la L-hydroxyproline, la L-isoleucine, la L-phénylalanine, ou une combinaison de ceux-ci.

16. Méthode selon la revendication 11, dans laquelle les matières formant une matrice comprennent en outre des éléments nutritifs, des vitamines, d'autres principes actifs, des édulcorants, des arômes, des colorants, des tensio-actifs, des agents de conservation, des antioxydants, des agents de viscosité, des minéraux, des composés de diagnostic, des engrais ou des insecticides.

17. Forme posologique solide à désintégration rapide portant des marques et / ou des caractères lisibles pouvant être obtenus par impression à jet d'encre selon les revendications 1 et 6 à 16.

18. Forme posologique selon la revendication 17, se désintégrant en 10 secondes au maximum dans de l'eau à 20 ºC.
